# EUROPEAN PATENT APPLICATION

(11) **EP 1 570 733 A1**
(43) Date of publication of application: **07.09.2005**
(21) Application number: 04004403.4
(22) Date of filing: 26.02.2004
(51) Int. Cl.: A01K 67/027

(54) **Mammal for studying DCIS and the effect of inactivated tumor suppressors**

(71) Applicant: Deppert, Wolfgang Willi, 22359 Hamburg (DE)
(72) Inventor: Deppert, Wolfgang Willi, 22359 Hamburg (DE)
(74) Representative: Schüssler, Andrea

(57) **Abstract**

The present invention relates to a mammal with inducible ductal carcinoma *in situ* (DCIS), the mammal containing (a) an oncogene which can be activated by lactotropic hormones and (b) a mutated tumor suppressor gene resulting in a tumor suppressor protein showing a partial or complete loss of activity. The invention also relates to a method of producing such a mammal and its use for studying (a) a DCIS or the progression thereof towards invasive ductal carcinoma of the breast and/or (b) the oncogenic potential of mutations of tumor suppressor genes, preferably p53, as well as for developing diagnostic and therapeutic means for the above purpose.

## Description

The present invention relates to a mammal in which a ductal carcinoma *in situ* (DCIS) of the female mammary gland can be induced. The invention also relates to a method of producing such a mammal and its use for studying (a) DCIS and/or its progression towards an invasive ductal carcinoma of the breast and/or (b) the oncogenic potential of mutations of tumor suppressor genes, preferably p53, and for the development of diagnostic and/or therapeutic means for the above purpose.

The mammary carcinoma which occurs with a frequency of about 10% among the female population is one of the pressing health problems of our time. About 80% of the mammary carcinomas are invasive ductal mammary carcinomas. Mammary tumors belonging to the type of ductal carcinoma *in situ* (DCIS) were frequently diagnosed in a mammography. DCIS is characterized by a non-invasive neoplastic proliferation, i.e. a proliferation not yet breaking through the basal membrane, of epithelial cells into the lumen of the ductulo-lobulary unit of the mammary system. A DCIS may develop into an invasive ductal mammary carcinoma. The molecular causes of this are, however, not known. Likewise, a prediction as to whether and when such a development occurs is not possible either. As a result, radical mastectomy, i.e. complete removal of the female breast and local lymph nodes, is usually carried out when DCIS of the female breast is diagnosed. Estimates, however, show that about 60% of the radical mastectomies represent an excessive treatment.

It is thus the object of the present invention to provide a product by which the molecular causes of DCIS, in particular

its progression towards an invasive ductal mammary carcinoma, can be studied and optionally possibilities for a reliable diagnosis and/or appropriate therapy can be shown.

According to the invention this is achieved by the subject matters defined in the claims.

The present invention is based on Applicant's insights that DCIS of the female mammary gland can be induced in mammals, e.g. mice, containing an oncogene, e.g. the early region of SV40, i.e. the gene for the SV40 T-Ag, which can be activated by lactotropic hormones such as estrogen, prolactin, insulin and hydrocortisone. He also found that DCIS may develop into an invasive ductal mammary carcinoma. Furthermore, it was found that by use of a mammal containing in addition to said oncogene a mutation within a tumor suppressor, preferably, p53, the study of the development of DCIS as well as the study of the oncogenic potential of an inactivated tumor suppressor can be improved. For example by use of the bi-transgenic mice the metastasing of DCIS can be better characterized.

According to the invention, Applicant's insights are used to provide a mammal having an inducible DCIS of the female mammary gland, which contains (a) an oncogene that can be activated by lactotropic hormones and (b) a mutated tumor suppressor gene resulting in a tumor suppressor protein showing a partial or complete loss of activity. DCIS may preferably develop into an invasive ductal mammary carcinoma.

The expression "DCIS of the female mammary gland" refers to a non-invasive neoplastic proliferation, i.e. a proliferation which does not yet break through the basal membrane, of epithelial cells into the lumen of the ductulo-lobulary unit of the mammary gland system. In particular, DCIS distinguishes itself by histological features, such as hyperchromatic, pleomorphous, large-scale structured or strikingly large nuclei. It may show a shifted nucleus-plasma relation or numerous mitotic patterns. DCIS may also be characterized in that the proliferation of the epithelial cells into the lumen of the duct manifests itself as a multi-layered or sievelike lining or as an intraluminal branching or by means of micropapillae. Moreover, DCIS may show as necroses, apoptosis patterns, psammoma bodies, i.e. onion skin-like crystallization products having calcifications, in the lumen of the duct and loss of the myoepithelial layer underneath the basal membrane.

The expression "lactotropic hormones" refers to hormones which are released by mammals, e.g. during pregnancy and/or lactation, and have a lactotropic effect. Examples of such hormones are estrogen, prolactin, insulin and hydrocortisone.

The expression "oncogene" comprises any gene or portions thereof which may have a cell-transforming property. Examples of such genes are erb A, erb B, fos, myc, E6, E7 and the early region of SV40, i.e. the gene for SV40 T-Ag, as well as mutated p53. The oncogene may also comprise sequences coding for a strong, i.e. immunodominant, T-cell epitope, e.g. the MHCI-restricted epitope n118 of the LCM virus nucleoprotein.

The expression "tumor suppressor" comprises any protein or portions thereof which are capable of suppressing malignant proliferation (directly or indirectly). Examples of such genes are well known to the person skilled in the art and comprise Smad4, Zac1, RB1, p53 (TP53) etc.

A "mutated tumor suppressor gene" encodes a tumor suppressor which has partially or completely lost its capability to suppress malignant proliferation. A preferred mutated tumor suppressor is p53 with mutations p53 245W (corresponding to human p53 248W) and p53 270H (corresponding to human p53 273H) being particularly preferred. Preferably, the non-human mammal of the present invention is homozygote for the mutated tumor suppressor gene.

The expression "oncogene that can be activated by lactotropic hormones" refers to the fact that the above oncogene can be activated by lactotropic hormones. This may be achieved in the most differing ways. It may be favorable for the oncogene to be controlled by a promoter which is specific to one or more lactotropic hormones. Such a promoter is e.g. the "whey acidic protein" (WAP) promoter. Its specificity comprises the lactotropic hormones estrogen, prolactin, insulin and hydrocortisone. Reference is made to the below description regarding the production of a mammal according to the invention.

The expression "mammal" comprises any animals, with the exception of humans, which release lactotropic hormones, e.g. during pregnancy and/or lactation, and which may contain an oncogene that can be activated by lactotropic hormones and a mutated tumor suppressor gene. Examples of such mammals are mice, rats, rabbits, horses, bovine animals, sheep, goats, monkeys, pigs, dogs and cats, mice being mentioned above all.

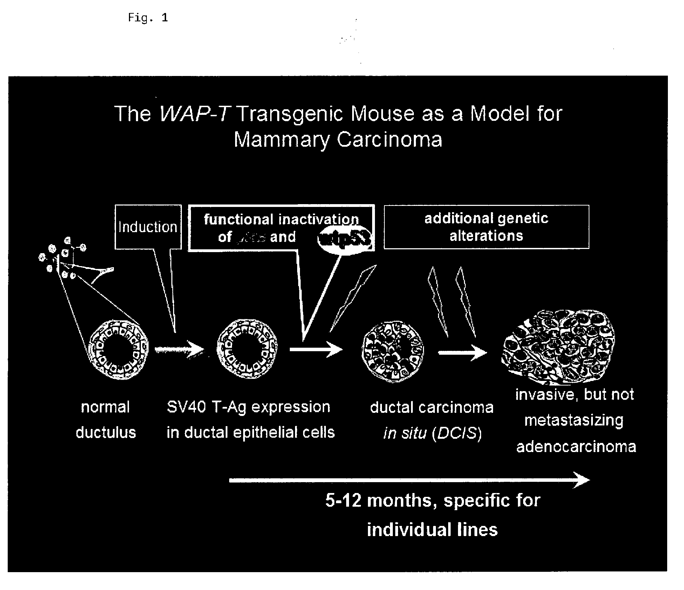

Preferred mammals of the present invention are mice which contain the gene for the SV40 T-Ag and the gene encoding the mutated tumor suppressor p53 under the control of the WAP promoter. The SV-40 T-Ag gene may also contain sequences coding for a strong, i.e. immunodominant, T-cell epitope, e.g. epitope n118 of the LCM virus nucleoprotein. For getting such mammals WAP-T or WAP-T-NP mice (Figure 1), particularly WAP-T-1, WAP-T-2, WAP-T-10, WAP-T-NP6, WAP-T-NP8 and WAP-T-NP10 mice are used for crossing with mice having a mutated tumor suppressor p53 gene. WAP-T or WAP-T-NP mice and methods for their production are in detail described in EP-A1 1 147 185 and WO 00/44889.

Another subject matter of the present invention relates to cells which are obtained from the mammal according to the invention. These cells may be present in any form, e.g. in a primary or long-term culture.

A mammal according to the invention may be provided by common methods. A method may be favourable which comprises the steps of:
(a) introducing a DNA coding for an oncogene into inseminated oocytes of a first mammal, the DNA being controlled by a promoter specific to lactotropic hormones;
(b) implanting the oocytes from (a) into pseudopregnant mammals; (c) selecting the progeny obtained in (b) for the formation of DCIS;
(d) introducing a DNA coding for a mutated suppressor gene under the control of a promoter into inseminated oocytes of a second mammal;
(e) implanting the oocytes from (d) into pseudopregnant mammals;
(f) selecting the progeny obtained in (e) for the presence of the mutated tumor suppressor gene;
(g) crossing the progeny of (c) and (f); and
(h) selecting the progeny obtained in (g) for the formation of DCIS and the presence of the mutated tumor suppressor gene.

As to the expressions "oncogene", "mutated tumor suppressor gene", "lactotropic hormones", "mammals" and "DCIS" reference is made to the above explanations.

Furthermore, the expression "pseudopregnant mammals" refers to mammals which were paired with non-potent, i.e. sterile or vasectomized, male mammals, and have a vaginal plug. Reference is made to the below example.

The expression "inseminated oocytes" refers to oocytes of pregnant mammals which have been emptied. Reference is made to the example of EP-A1 147 185 and WO 00/44889, respectively.

The expression "DNA coding for an oncogene and controlled by a promoter specific to lactotropic hormones" relates to a DNA present in any form and having these properties. The DNA may be present as such or in combination with another DNA, e.g. a vector. It may also be circular or linear. Furthermore, it may contain sequences supporting a recombination with the DNA of the mammal. In addition, it may contain sequences which code for a T-cell epitope, e.g. the MHC I restricted epitope n118 of the LCM virus nucleoprotein. Such a DNA was deposited with DSMZ (*Deutsche Sammlung von Mikroorganismen und Zellkulturen*) [German-type collection of microorganisms and cell cultures] as pWAP-T-NP under DSM 12608 on December 22, 1998.

The person skilled in the art also knows conditions and materials to carry out steps (a) - (h). As to the selection in (c) and (f) he will use e.g. methods by means of which the above-mentioned histologic features can be detected.

A mammal is provided by the present invention, in which DCIS can be induced. Furthermore, an invasive ductal mammary carcinoma can develop from DCIS. The molecular causes of DCIS, its progression towards an invasive ductal mammary carcinoma or its potential for metastasising may thus be studied. In particular, it is of advantage that mammals can be provided which have differently long latent periods until DCIS or an invasive ductal carcinoma has been developed, so that by means of a comparative study a correlation can be made between DCIS type (including the identified molecular markers) and the risk of DCIS. It is also of advantage that the role of the immune system in the development of DCIS or its progression towards an invasive ductal mammary carcinoma can be studied, which is supported by the presence of a strong T-cell epitope in the oncogene product. Besides, the present invention provides a basis for the development of diagnostic markers by which individual development levels of DCIS or the invasive ductal carcinoma can be detected and thus predictions can be made regarding the development of DCIS, its progression or its metastasising potential. The present invention also provides the possibility of developing therapeutic agents against the above diseases.

### Brief description of the drawings

- **Figure 1**: shows a scheme as regards a WAP-T transgenic mice model for mammary carcinoma.
- **Figure 2**: shows the DNA used for the production of an inducible mutant p53 mouse. The DNA is used in a linear form and injected into inseminated oocytes.

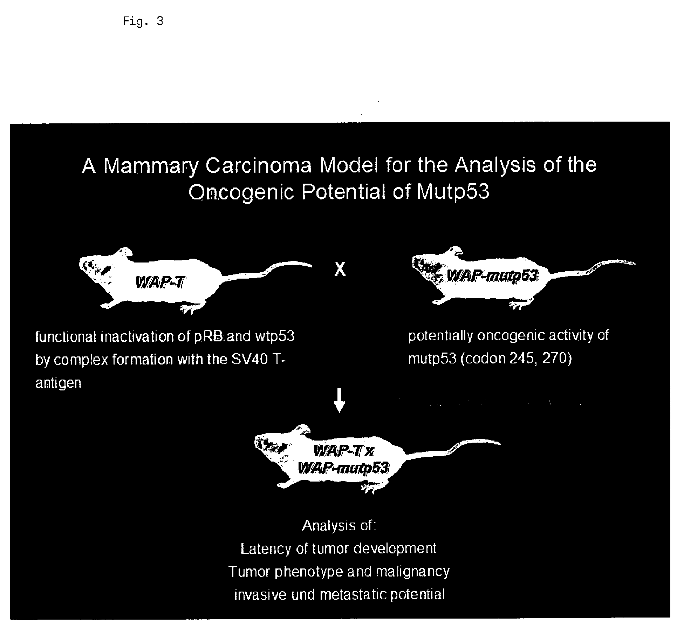

- **Figure 3**: is a scheme showing the crossing of a WAP-T mouse and a WAP-mutp53 mouse and possible uses of the WAP-T x WAP-mutp53 mouse of the present invention.

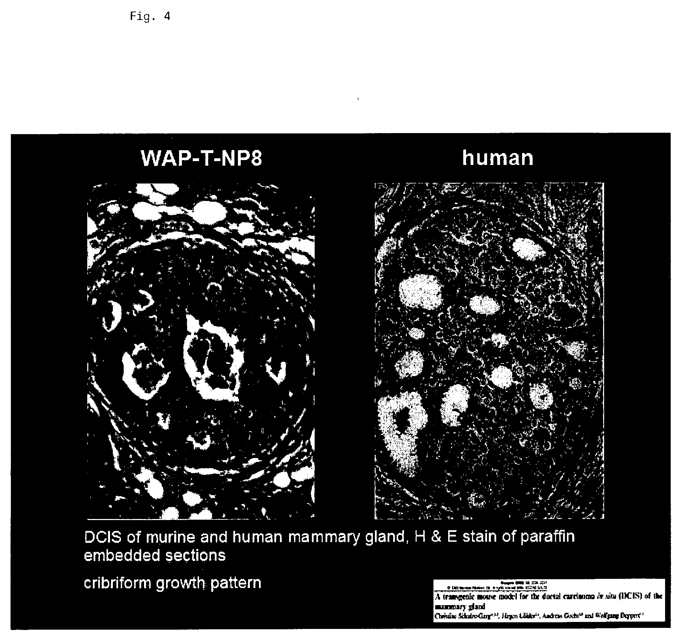

- **Figure 4**: shows H & E stains of paraffin embedded sections of DCIS of murine (WAP-T-NP8) and human mammary gland.

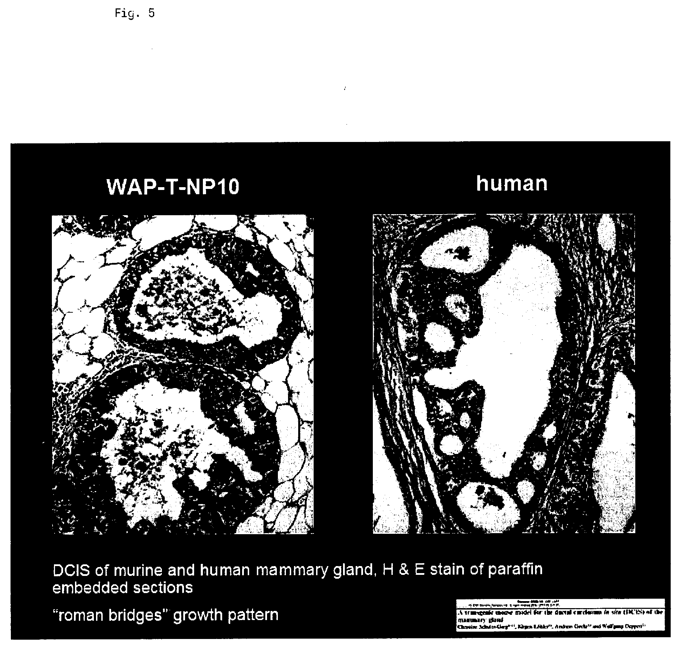

- **Figure 5**: shows DCIS of murine (WAP-T-NP10) and human mammary gland.

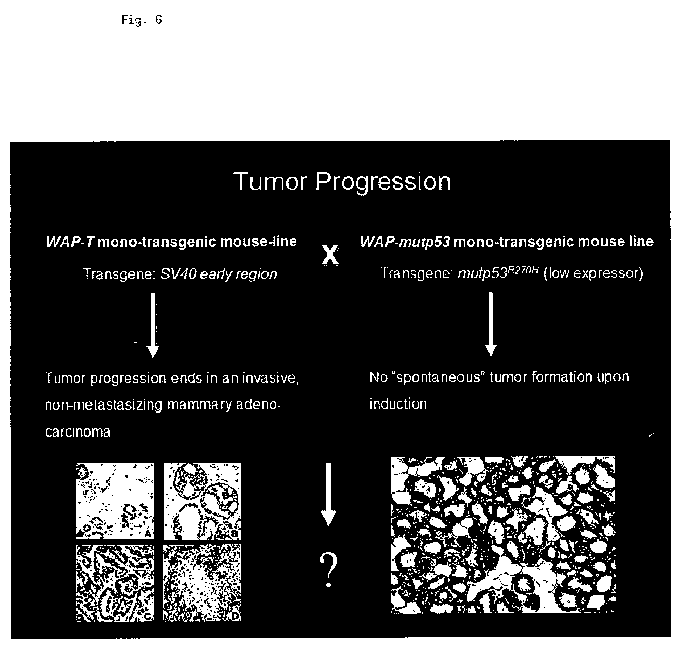

- **Figure 6**: shows a comparison of tumor progression in WAP-T mono-transgenic mice vs. WAP-mutp53 mono-transgenic mice.
- **Figure 7**: is a Northern-Blot showing the tissue specific expression of transgenes (T-antigen; mutp53^{R270H}) in WAP-T x WAP-mutp53 bi-transgenic mice of the present invention.
- **Figure 8**: shows a comparison of the immunostaining of a WAP-T mouse (immunostaining for T-antigen) vs. a WAP-T x WAT-mutp53 mouse of the present invention (immunostaining for mutp53).
- **Figure 9**: shows a comparison of the invasion of the tumor into the muscle in a DCIS mouse WAP-T vs. a WAP-T x WAT-mutp53 mouse of the present invention.
- **Figure 10**: shows the invasion of tumor cells into the connective tissue in a WAP-T x WAT-mutp53 mouse of the present invention (cytokeratine staining).
- **Figure 11**: shows the infiltration of the tumor cells into a lymph node in a WAP-T x WAT-mutp53 mouse of the present invention.
- **Figure 12**: shows the invasion of tumor cells into the blood and lymphatic system of a WAP-T x WAT-mutp53 of the present invention.
- **Figure 13**: shows the metastasis in the spleen of a WAP-T x WAT-mutp53 mouse of the present invention.
- **Figure 14**: shows the metastasis in the lung with blood vessel of a WAP-T x WAT-mutp53 mouse of the present invention.

The invention is explained by the following example.

### Example: Production of a mammal according to the invention

### (A) Generation of WAP-T-mice

WAP-T-mice were generated as described in EP-A1 147 185 and WO 00/44889. Examples of these mice are characterized as follows:

### WAP-T-1

These mice usually develop multifocal invasive ductal mammary carcinoms after an average of 7 months following the induction with lactotropic hormones. No preferential tumor formation can be observed in one of the mammae. Invasive carcinomas are, as a rule, differentiated tubularly to papillarily and have partially solid anaplastic portions which may also show a desmoplastic reaction. Pulmonary metastases occur occasionally. However, the majority of animals does not form metastases, and the primary tumors only grow slowly. Macroscopically non-affected mammae show multifocal DCIS. Due to the isomorphous manifestation of the nuclei most DCIS may be classified as "low grade" in analogy to the classification of human DCIS according to the NUYS index. Comedonecroses and psammoma bodies are observed from time to time. However, there is a formation of intraluminal branches ("roman arches" formation).

### WAP-T-2

These animals have a light-brown coat. Furthermore, they develop invasive ductal mammary carcinomas about 6 months after the induction with lactotropic hormones. Differentiated mammary carcinomas are predominantly tubular to lobular. Anaplastic mammary carcinomas appear with some tumor giant cells. Micrometastases in lymph nodes are observed. In rare cases, fibrosarcomas may form, starting from mammary or uterus. The multifocally occurring DCIS show micropapillary and cribriform growth patterns. Forms which have monolayered lining and comdeonecrosis or psammoma bodies are also observed. Since the cell nuclei are usually isomorphous, these DCIS cannot be assessed as "high grade" (cf. above).

### WAP-T-10

About 8 months after the induction with lactotropic hormones, these mice develop palpable ductal mammary carcinomas which may form metastases. Both solid carcinomas and carcinomas with little differentiation which have numerous mitoses as well as tubular to papillary forms may be found. The investigated metastases are differentiated papillarily. The multifocally occurring DCIS have comedonecroses and due to the morphology of nuclei (pleomorphism, hyperchromasia, and others) correspond to a "high grade" DCIS.

### WAP-T-NP6

These mice develop palpable invasive carcinomas about 11 months after multiple induction with lactotropic hormones. In rare cases, hepatocellular adenomas and adenomas of the salivary gland occur as well. The mammary carcinomas are differentiated predominantly tubularly or papillarily, in some cases they are differentiated only moderately with extensive necroses and they are partially also solid. Micrometastases occur in mammary lymph nodes. The cell nuclei of the multifocally occurring DCIS are usually inconspicuously "low grade" (see above) and comedonecroses are present.

### WAP-T-NP8

These mice develop invasive ductal carcinomas about 5 months following induction with lactotropic hormones. Both tubulo-papillarily differentiated and poorly differentiated solid tumors are found. Animals having poorly differentiated tumors show pulmonary metastases. The lumens of localized small invasive carcinomas and of DCIS are in some cases infiltrated by granulocytes. Non-epithelial tumors of mammary origin, e.g. fibrosarcomas and osteosarcomas, and infiltrating histiocytic sarcomas also occur occasionally. The multifocal DCIS apparent 15 to 20 weeks following induction with lactotropic hormones correspond to a "high grade" form due to the morphology of cell nuclei.

### WAP-T-NP10

These mice develop an invasive ductal mammary carcinoma about 11 months following several inductions with lactotropic hormones. These carcinomas are frequently differentiated tubularly to papillarily and have solid and necrotic but only moderately differentiated portions. The DCIS appear with isomorphous nuclei (not "high grade"; see above). DCIS having micropapillary growth and forms with total loss of the myoepithelial layer and psammoma body formation occur.

### (B) Generation of inducible mutant p53 transgenic mice

About 20 female CB6F1 mice at the age of 4 to 5 weeks were superovulated by intraperitoneal injection of 5 U PMS (pregnant mare's serum) on day 1 and another intraperitoneal injection of 5 U hCG (human chorionic gonadotropin) on day 3 and were paired with male CB6F1 animals in the evening of that very day. In the morning of the 4^{th} day, the animals were investigated for the presence of a vaginal plug, positive animals were sacrificed by cervical dislocation and the oviducts were removed. The oocytes were removed from the oviducts and placed into M2 medium, the cumulus cells were separated by short incubation using hyaluronidase, the oocytes were washed thoroughly and stored in an incubator (5 % CO₂, 85 % humidity, 37°C) in M16 medium covered with paraffin oil until they were microinjected.

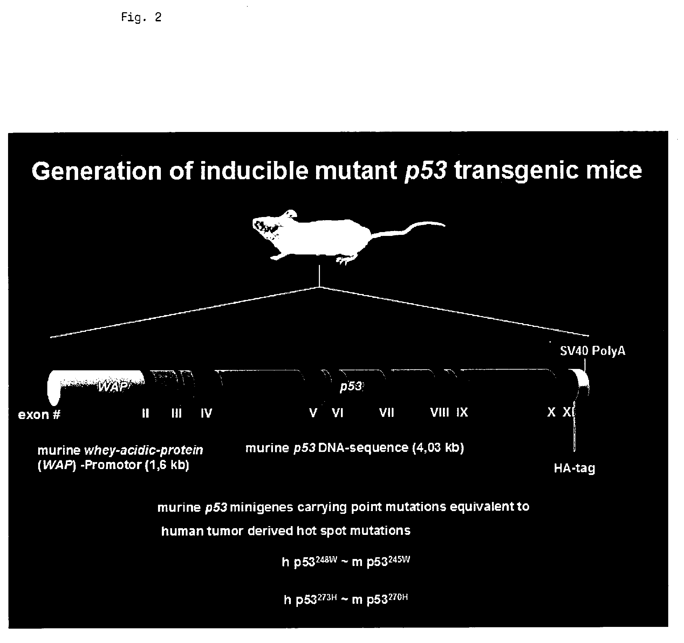

The DNA shown in Figure 2 was injected into the male pronucleus of inseminated oocytes on the 4^{th} day. Injected oocytes were then incubated in the incubator up to the retransfer taking place the following day. For providing pseudopregnant foster mice, about 25 female B6CBAF1 mice at the age of 8 to 12 weeks were paired with vasectomized male mice in the evening before the microinjection. In the morning of the 5^{th} day, the animals with vaginal plug were selected for retransfer of the injected oocytes. The microinjected oocytes cultured overnight and proliferated to the two-cell stage were reimplanted on the 5^{th} day. In this connection, 10-15 embryos were rinsed into the infundibulum of an oviduct of a narcotized foster mouse. 19-20 days after the retransfer the implanted embryos were born.

### (C) Generation of WAP-T x WAP-mutp53 mice of the present invention

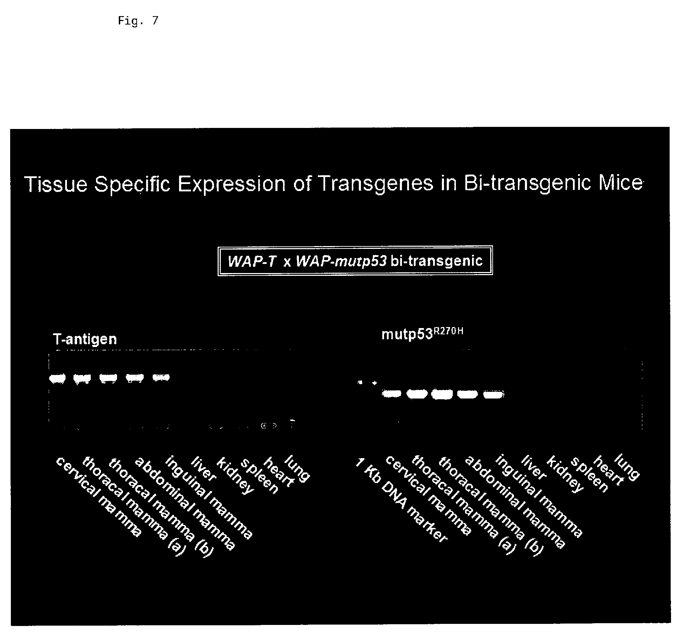

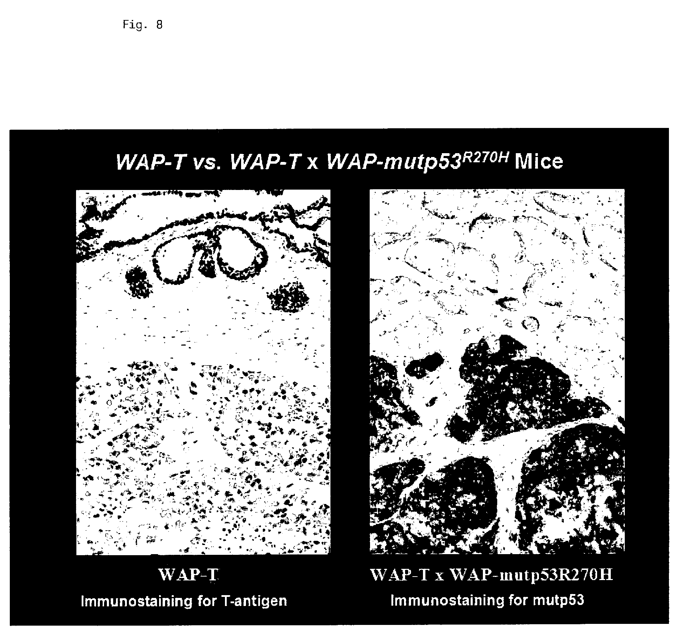

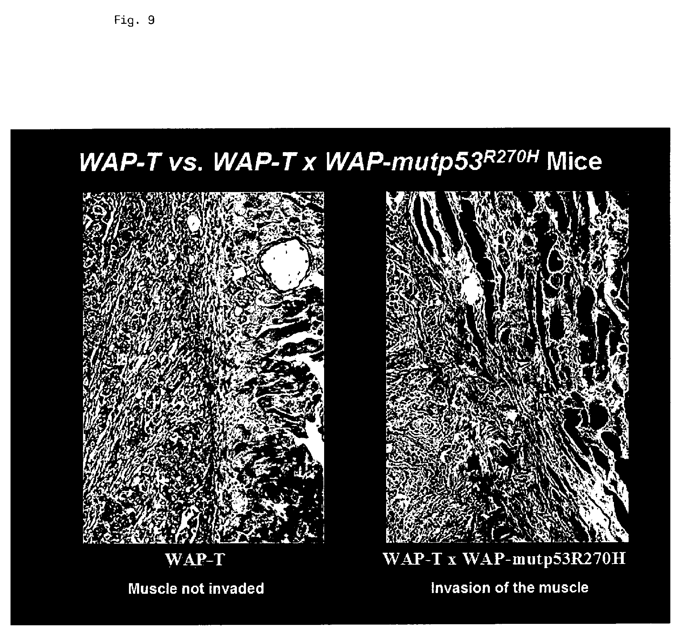

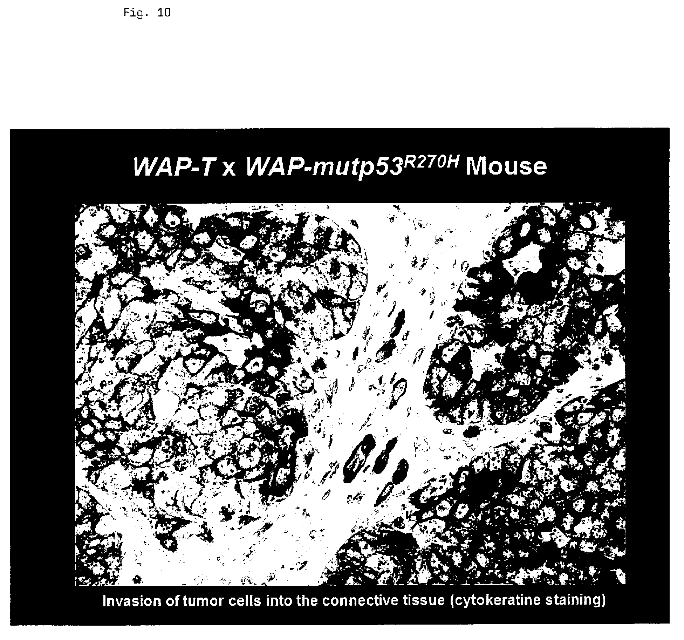

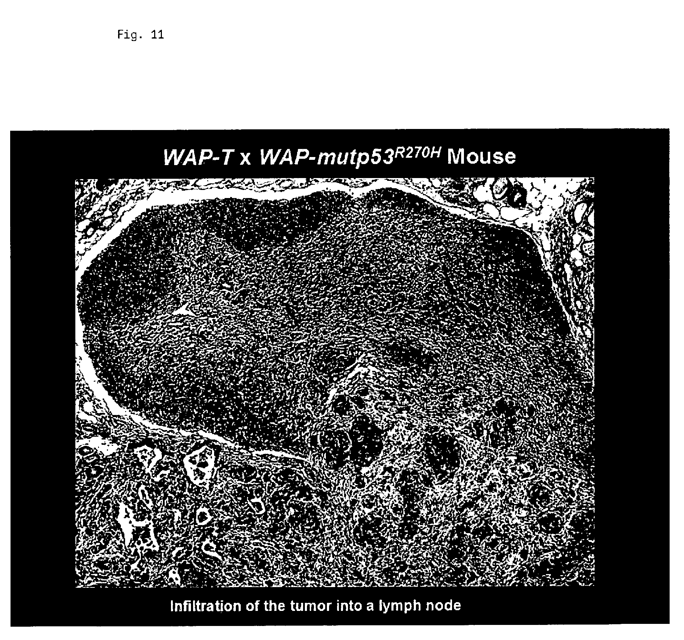

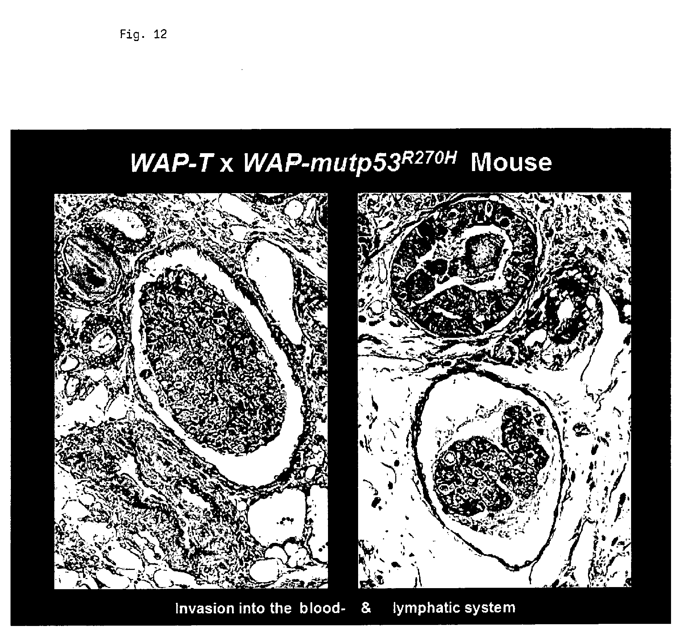

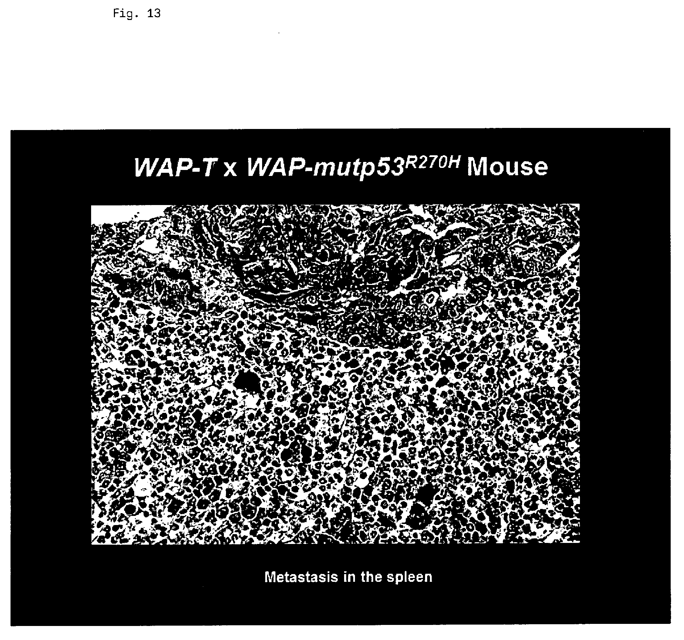

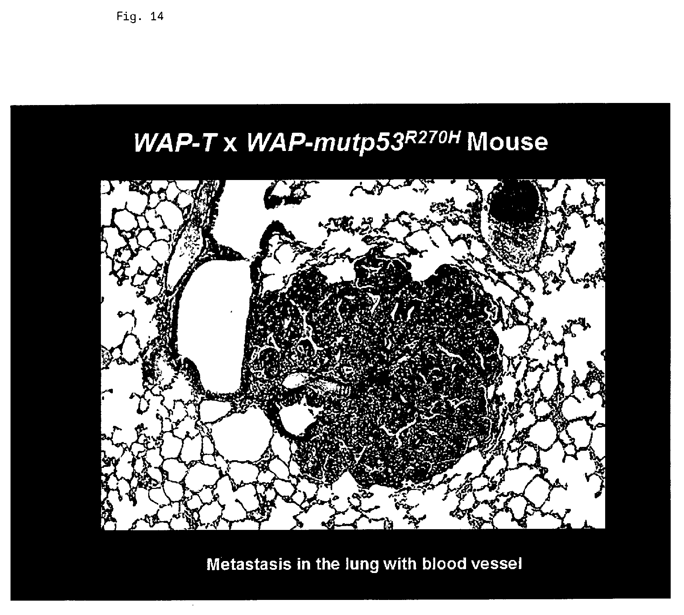

Progeny of the two types of mice described in (A) and (B) were crossed and mice having the desired genotype were selected according to routine methods. One mouse line, i.e., WAP-T1 x mutp53 270H was further characterized as shown in Figures 7 to 14. From the results presented in these Figures it is apparent that the bi-transgenic mammals of the present invention offer several advantages compared to the mono-transgenic WAP-T mice since mutp53 exerts a profound effect on tumor progression:

The bi-transgenic mice show increased malignancy compared to WAP-T mono-transgenic mice, i.e. (a) invasion into connective tissue, into lymph nodes, into the blood system and into the muscle, and (b) metastasis of the lung and spleen.

## Claims

1. A non-human mammal with inducible ductal carcinoma *in situ* (DCIS), wherein the mammal contains (a) an oncogene that can be activated by lactotropic hormones and (b) a mutated tumor suppressor gene.

2. The non-human mammal according to claim 1, wherein DCIS develops into an invasive ductal mammary carcinoma.

3. The non-human mammal according to claim 1 or 2, wherein the oncogene comprises a sequence coding for a strong T-cell epitope.

4. The non-human mammal according to any of claims 1 to 3, wherein the oncogene is a gene coding for SV40 TAg.

5. The non-human mammal according to claim 3 or 4, wherein the sequence codes for the n118 epitope of the LCM virus nucleoprotein.

6. The non-human mammal according to any of claims 1 to 5, wherein the lactotropic hormones comprise estrogen, prolactin, insulin and hydrocortisone.

7. The non-human mammal according to any of claims 1 to 6, wherein the mutated suppressor gene is mutp53.

8. The non-human mammal according to any one of claims 1 to 7, wherein the oncogene and/or the mutated tumor suppressor gene is controlled by the WAP promoter.

9. The non-human mammal according to any one of claims 1 to 8, which is a mouse or rat.

10. The non-human mammal of claim 9 that carries the mutation p53 245W and/or p53 270H.

11. A method of providing a mammal according to any of claims 1 to 10, comprising the steps of:
(a) introducing a DNA coding for an oncogene into inseminated oocytes of a first mammal, the DNA being controlled by a promoter specific to lactotropic hormones;
(b) implanting the oocytes from (a) into pseudopregnant mammals; (c) selecting the progeny obtained in (b) for the formation of DCIS;
(d) introducing a DNA coding for a mutated suppressor gene under the control of a promoter into inseminated oocytes of a second mammal;
(e) implanting the oocytes from (d) into pseudopregnant mammals;
(f) selecting the progeny obtained in (e) for the presence of the mutated tumor suppressor gene;
(g) crossing the progeny of (c) and (f); and
(h) selecting the progeny obtained in (g) for the formation of DCIS and the presence of the mutated tumor suppressor gene.

12. Use of the mammal according to any of claims 1 to 10 or the mammal obtained according to the method of claim 11 for studying DCIS, its progression towards an invasive ductal carcinoma and/or the metastasising potential of DCIS.

13. Use of the mammal according to any of claims 1 to 10 or the mammal obtained according to the method of claim 11 for the research and development of diagnostic markers and therapeutic agents for a DCIS, an invasive ductal carcinoma or metastasis.

14. Use of the mammal according to any of claims 1 to 10 or the mammal obtained according to the method of claim 11 for studying the oncogenic potential of the mutated tumor suppressor gene.
